# EUROPÄISCHE PATENTANMELDUNG

(11) **EP 0 732 393 A1**
(43) Veröffentlichungstag der Anmeldung: **18.09.1996**
(21) Anmeldenummer: 96104162.1
(22) Anmeldetag: 15.03.1996
(51) Int. Cl.: C11C 1/00, C11B 11/00, C07C 59/01, C07C 51/43

(54) **Verfahren zur Fraktionierung von Wollwachssäuregemischen**

(30) Priorität: 17.03.1995 DE 19509760
(71) Anmelder: SKW Trostberg Aktiengesellschaft, D-83308 Trostberg (DE)
(72) Erfinder: Heidlas, Jürgen, Dr., 83308 Trostberg (DE); Ober, Martin, 83352 Altenmarkt (DE); Cully, Jan, Dr., 84518 Garching (DE)
(74) Vertreter: Huber, Bernhard, Dipl.-Chem.

(57) **Zusammenfassung**

Zur Fraktionierung von Wollwachssäuregemischen durch Lösen des Ausgangsmaterials in einem polaren organischen Lösemittel (-gemisch) behandelt man das gelöste Wollwachssäuregemisch bei einer Temperatur zwischen -5 und 30°C und einem Druck zwischen 10 und 70 bar mit gasförmigem Kohlendioxid und trennt die hierbei als Feststoff anfallenden Hydroxyfettsäuren aus der Lösung ab.

## Beschreibung

Die vorliegende Erfindung betrifft ein Verfahren zur Fraktionierung von Wollwachssäuregemischen, das es ermöglicht, die Fraktion der Hydroxyfettsäuren von der Fraktion der nicht hydroxylierten Fettsäuren abzutrennen und damit die Hydroxyfettsäuren als kristallines Produkt und in hoher Reinheit zu gewinnen.

Aus natürlichem Wollwachs (Lanolin) kann eine chemisch uneinheitliche Fraktion von längerkettigen Carbonsäuren gewonnen werden, die im wesentlichen aus verzweigten und unverzweigten Fettsäuren sowie Hydroxyfettsäuren, vor allem alpha-Hydroxyfettsäuren, besteht (Motiuk, K.; J. Am. Oil Soc. 56, 91 (1979)). Das Interesse an geeigneten Verfahren, die es ermöglichen, das komplexe Säuregemisch zu fraktionieren, hat sich verstärkt, seit die kosmetische und pharmazeutische Industrie die unterschiedlichen Anwendungsbereiche der nach chemischen Klassen aufgetrennten Fraktionen zunehmend nutzt. Besonders vorteilhaft ist in diesem Zusammenhang die toxikologische Unbedenklichkeit von Wollwachs und dessen Bestandteilen, insbesondere bei deren topischer Anwendung beim Menschen (J. Environ. Path. Tox. 4, 63-92 (1980)).

In der älteren Literatur sind zahlreiche Verfahrensweisen beschrieben, mit denen Wollwachssäuregemische aufgetrennt werden können. Diese Verfahren beruhen auf den Prinzipien der Destillation, der Kristallisation oder der Lösemittelextraktion, wobei jedoch alle diese Verfahren mit erheblichen Nachteilen behaftet sind. Bei destillativen Verfahren besteht generell die Gefahr, daß das Produkt durch eine zu starke thermische Belastung unerwünschten Reaktionen unterliegt, wie z.B. Lactidbildung zwischen zwei alpha-Hydroxysäuremolekülen oder Dehydratisierungsreaktionen, die letztendlich zu hohen Ausbeuteverlusten führen.

Bei Verfahren der Kristallisation wurde oftmals mit Lösemittelgemischen gearbeitet, wobei zum einen relativ lange Kristallisationszeiten in Kauf genommen werden mußten, die unter wirtschaftlichen Gesichtspunkten schlechte Raum-Zeit-Ausbeuten bedingen, und mit denen zum anderen oftmals eine nur unzureichende Trennung erzielt werden konnte. Jüngst wurde in der EP 0 555 776 A1 ein neues Lösemittelverfahren zur Fraktionierung von Wollwachssäuregemischen offengelegt, das mindestens zwei Extraktionsschritte mit organischen Lösemitteln unterschiedlicher Polarität empfiehlt, um eine hinreichende Trennung und Reinheit der Säurefraktionen zu erhalten. Zur Erreichung einer hohen Produktreinheit muß dieses Verfahren unter Zuhilfenahme von Aussalzungsschritten ggf. mehrfach durchlaufen werden. Abgesehen vom großen Zeitaufwand, der im Bereich mehrerer Stunden liegt, kann bei der Durchführung des Verfahrens im größeren Maßstab technologisch das Problem auftreten, daß die ungelösten Fettsäurefraktionen, die oftmals eine wachsartige und damit hochviskose Konsistenz aufweisen, bei der Filtration die Filtersysteme verstopfen. Das Verfahrensziel, die Hydroxyfettsäuren in möglichst reiner und kristalliner Form zu erhalten, kann mit dieser Verfahrensweise nur sehr umständlich und mit technologischen Problemen behaftet erreicht werden.

Aufgabe der Erfindung ist es daher, ein Verfahren zur Fraktionierung von Wollwachssäuregemischen zu schaffen, mit dem die genannten Nachteile der bekannten Verfahren vermieden werden und mit dem das Ausgangsmaterial effizient in hydroxylierte und nicht hydroxylierte Fettsäurefraktionen großer Reinheit aufgetrennt werden kann.

Die Aufgabe wird erfindungsgemäß dadurch gelöst, daß man das in einem polaren Lösemittel(-gemisch) gelöste Wollwachssäuregemisch bei Temperaturen zwischen -5 und 30°C und einem Verfahrensdruck zwischen 10 und 70 bar einer Druckbehandlung mit gasförmigem Kohlendioxid unterzieht und die hierbei als Feststoff anfallenden Hydroxyfettsäuren abtrennt.

Überraschenderweise hat sich dabei gezeigt, daß sich das Expansionsverhalten des jeweiligen Lösemittels durch das Eindrücken von gasförmigem Kohlendioxid gezielt so steuern läßt, daß bevorzugt die Hydroxyfettsäuren in kristalliner Form ausfallen und deshalb überraschend leicht und in sehr guter Qualität abgetrennt werden können.

Erfindungsgemäß werden zum Lösen des Ausgangsmaterials polare organische Lösemittel mit bis zu 5 C-Atomen aus der Reihe der kurzkettigen Ester, wie insbesondere Ethylacetat, der Ketone, wie insbesondere Aceton, und der Alkohole, wie Methanol und Ethanol, oder Mischungen dieser Lösemittel eingesetzt. Das zu fraktionierende Wollwachssäuregemisch wird in diesen Lösemitteln bevorzugt in einer Konzentration zwischen 10 und 50 Gew.-%, besonders bevorzugt zwischen 20 und 40 Gew.-%, und bei Raumtemperatur, also vorzugsweise zwischen 15 und 25°C, gelöst, wofür nur wenige Minuten benötigt werden.

Die eigentliche Fraktionierung des so gelösten Gemisches wird anschließend in der Weise ausgeführt, daß dem Lösemittel(-gemisch) bei einer Temperatur zwischen -5 und 30°C gasförmiges Kohlendioxid aufgedrückt und in diesem Zustand über einen Zeitraum von maximal 1 Stunde gehalten wird. Durch das Aufdrücken des Gases expandiert das Volumen des Lösemittels, wobei die Hydroxyfettsäurefraktion ausfällt. Die Fällung wird durch Stehenlassen unter gleichen Bedingungen vervollständigt. Zweckmäßigerweise wird das Verfahren in einem temperierbaren Druckgefäß durchgeführt. Der maximale Verfahrensdruck wird bei gegebener Temperatur durch den Druck limitiert, bei dem das Kohlendioxidgas sich zu verflüssigen beginnt, der aber bei der Fraktionierung gemäß vorliegendem Verfahren nicht ganz erreicht wird. Bei der maximalen Verfahrenstemperatur von 30°C erreicht dieser Druck seinen Maximalwert, nämlich ca. 70 bar; bei der minimalen Verfahrenstemperatur von -5 °C liegt der Druck bei ca. 25 bar. Nach einer Haltezeit von 5 bis 60 Minuten, die der Vervollständigung der Ausfällung dient, wird unter Aufrechterhaltung des Verfahrensdruckes das Sediment, das überwiegend aus dem Hydroxyfettsäuregemisch besteht, von der Lösung abgetrennt. Dieser Separationsschritt wird gemäß einer bevorzugten Ausführungsform verfahrenstechnisch äußerst vorteilhaft über eine Filterplatte am Boden des Druckgefäßes vor dem Auslaß durchgeführt, über die die Lösung abgezogen und das Sediment abfiltriert wird. Die im wesentlichen in der Lösung verbleibenden nicht hydroxylierten Fettsäuren lassen sich aus dem Filtrat isolieren, vorzugsweise indem nach dem Separationsschritt das organische Lösemittel(-gemisch), ggf. unter reduziertem Druck, abdestilliert wird.

Die Ausbeute und Reinheit der ausgefällten Hydroxyfettsäuren kann erfindungsgemäß über die Parameter Temperatur und Kohlendioxiddruck und in Abhängigkeit vom gewählten Lösemittel(gemisch) beeinflußt werden. Entsprechend der Zielsetzung des Verfahrens kann die Trennung des Gemisches deshalb mit diesen Verfahrensparametern gezielt gesteuert werden.

Bei einer weiteren bevorzugten Ausführungsform des erfindungsgemäßen Verfahrens werden die als kristallines Sediment angefallenen Hydroxyfettsäuren mit flüssigem Kohlendioxid gewaschen, wodurch Lösemittelreste nahezu vollständig entfernt werden können und vor allem die geruchliche Qualität des Produktes stark verbessert wird. Dazu wird das Kohlendioxid bei unterkritischen Temperaturen von < 30 °C und bei Drücken < 100 bar, also im flüssigen Zustand, eingesetzt. Die Löslichkeit der Hydroxyfettsäuren im flüssigen Kohlendioxid ist unter diesen Zustandsbedingungen nur sehr gering, weshalb durch den Waschvorgang kaum Ausbeuteverluste an Hydroxyfettsäuren verursacht werden. Abhängig vom Grad der angestrebten geruchlichen Verbesserung und der Qualität des Ausgangsmaterials kann die Spülmenge an flüssigem Kohlendioxid in relativ weiten Bereichen variiert werden; sie beträgt zweckmäßig 0,5 bis 100 g Kohlendioxid pro g Hydroxyfettsäure.

Durch dieses Nachwaschen des Filterrückstandes mit flüssigem Kohlendioxid gelingt es, die Reinheit der Hydroxyfettsäuren zusätzlich zu steigern, Lösemittelreste mit dem Ziel der Produkttrocknung zu entfernen und zugleich den unerwünschten Geruch des Ausgangsmaterials im wesentlichen zu beseitigen.

Die entscheidenden Vorteile des erfindungsgemäßen Verfahrens gegenüber den bekannten Verfahren sind demzufolge darin zu sehen, daß die Hydroxyfettsäuren als feinkristallines Produkt anfallen, das gut filtrierbar ist, und daß die Verfahrenszeit vergleichsweise kurz gehalten werden kann, so daß gute Raum/Zeit-Ausbeuten erreicht werden können; zudem kann das Verfahren so temperaturschonend durchgeführt werden, daß eine thermische Schädigung der Produkte ausgeschlossen ist.

Die nachfolgenden Beispiele sollen diese Vorteile des erfindungsgemäßen Verfahrens verdeutlichen.

### Beispiele

Die Verfahrensbeispiele wurden in einem Hochdruckautoklaven mit Sichtfenster und Mantelkühlung (Autoklavenvolumen 400 ml) durchgeführt. Am Boden des Autoklaven ist vor einem Ausgang eine Metallsinterplatte angeordnet, über die das die Hydroxyfettsäuren enthaltende Sediment abfiltriert werden kann. Das eingesetzte Wollwachssäuregemisch (WWS) hatte eine Hydroxyzahl von 60.

| Beispiel | 1 | 2 | 3 |
|---|---|---|---|
| Lösemittel | Aceton | Ethylacetat | Ethanol |
| Lösemittelvolumen (ml) | 50 | 50 | 50 |
| WWS in Lösung (Gew.-%) | 25 | 25 | 25 |
| CO₂-Druck (bar) | 30 | 28 | 30 |
| Temperatur bei Druckbehandlung (°C) | 0 | -5 | 0 |
| Zeit zur Sedimentation (Min) | 45 | 45 | 30 |
| relative Ausbeute an Sediment (% vom WWS) | 21 | 17 | 18 |
| OH-Zahl des Filtratrückstandes | 190 | 182 | 210 |
| OH-Zahl des Eluates | 26 | 35 | 27 |
| Nachwaschen mit CO₂ | - | - | + |
| Temperatur (°C) | - | - | 25 |
| Druck (bar) | - | - | 75 |
| Spülmenge CO₂ (g/g Hydroxyfettsäuregemisch) | - | - | 3 |

## Patentansprüche

1. Verfahren zur Fraktionierung von Wollwachssäuregemischen durch Lösen des Ausgangsmaterials in einem polaren organischen Lösemittel(-gemisch),
**dadurch gekennzeichnet,**
daß man das gelöste Wollwachssäuregemisch bei einer Temperatur zwischen -5 und 30°C und einem Druck zwischen 10 und 70 bar mit gasförmigem Kohlendioxid behandelt und die hierbei als Feststoff anfallenden Hydroxyfettsäuren aus der Lösung abtrennt.

2. Verfahren nach Anspruch 1,
**dadurch gekennzeichnet,**
daß man als polares Lösemittel Ester, Ketone und Alkohole mit bis zu 5 C-Atomen oder Mischungen davon einsetzt.

3. Verfahren nach Anspruch 2,
**dadurch gekennzeichnet,**
daß man als polares Lösemittel Ethylacetat, Aceton, Methanol, Ethanol oder Mischungen davon verwendet.

4. Verfahren nach einem der Ansprüche 1 bis 3,
**dadurch gekennzeichnet,**
daß man das Wollwachssäuregemisch dem organischen Lösemittel im Konzentrationsbereich von 10 bis 50 Gew.-%, bevorzugt 20 bis 40 Gew.-%, zusetzt.

5. Verfahren nach einem der vorhergehenden Ansprüche,
**dadurch gekennzeichnet,**
daß man das Wollwachssäuregemisch dem organischen Lösemittel bei Temperaturen zwischen 15 und 25°C zusetzt.

6. Verfahren nach einem der vorhergehenden Ansprüche,
**dadurch gekennzeichnet,**
daß man das Hydroxyfettsäuregemisch vom Lösemittel(-gemisch) abfiltriert.

7. Verfahren nach einem der vorhergehenden Ansprüche,
**dadurch gekennzeichnet,**
daß man das abgetrennte Hydroxyfettsäuregemisch mit flüssigem Kohlendioxid wäscht.

8. Verfahren nach Anspruch 7,
**dadurch gekennzeichnet,**
daß man das Nachwaschen mit flüssigem Kohlendioxid bei Temperaturen < 30°C und Drücken < 100 bar durchführt.

9. Verfahren nach Anspruch 7 oder 8,
**dadurch gekennzeichnet,**
daß man pro g Hydroxyfettsäuregemisch 0,5 bis 100 g flüssiges Kohlendioxid einsetzt.

10. Verfahren nach einem der vorhergehenden Ansprüche,
**dadurch gekennzeichnet,**
daß man es in einem temperierbaren Druckgefäß mit einer Filterplatte, die vor dem Auslaß angeordnet ist, durchführt.

11. Verfahren nach einem der vorhergehenden Ansprüche,
**dadurch gekennzeichnet,**
daß man die nicht hydroxylierten Fettsäuren aus der filtrierten Lösung durch Entfernen des Lösemittels gewinnt.
